# EUROPÄISCHE PATENTANMELDUNG

(11) **EP 4 603 483 A1**
(43) Veröffentlichungstag der Anmeldung: **20.08.2025**
(21) Anmeldenummer: 24157569.5
(22) Anmeldetag: 14.02.2024
(51) Int. Cl.: C07D 323/06

(54) **VERFAHREN ZUR HERSTELLUNG VON TRIOXANDERIVATEN**

(71) Anmelder: Evonik Oxeno GmbH & Co. KG, 45772 Marl (DE)
(72) Erfinder: FRANKE, Robert, 45772 Marl (DE); SCHNEIDER, Carolin, 40789 Monheim am Rhein (DE); JACKSTELL, Ralf, 18106 Rostock (DE); BELLER, Matthias, 18211 Ostseebad Nienhagen (DE)
(74) Vertreter: Evonik Patent Association

(57) **Zusammenfassung**

Verfahren zur Herstellung von Trioxanderivaten.

## Beschreibung

Die vorliegende Erfindung betrifft ein Verfahren zur Herstellung von Trioxanderivaten.

Der vorliegenden Erfindung lag die Aufgabe zugrunde, ein Verfahren zur Herstellung von Trioxanderivaten bereitzustellen. Hierbei soll eine gute Ausbeute erzielt werden.

In US 2017/0233366 A1 wird ein Verfahren zur Herstellung von Trioxan beschrieben. Hierbei wird ein wässrige Formaldehyd-Lösung in Gegenwart von Methansulfonsäure bei 105 °C zu Trioxan umgesetzt. Entsprechend Tabelle 1 wurde ein Umsatz an Formaldehyd von 40 % erzielt.

Diese Aufgabe wird gelöst durch ein Verfahren gemäß Anspruch 1.

Verfahren umfassend die Verfahrensschritte:
a) Vorlegen eines Esters gemäß der Formel (**I**): 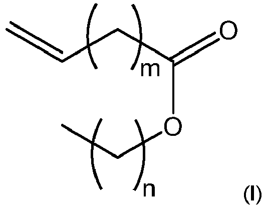 wobei m für eine ganze Zahl von 1 bis 10 steht und n für eine ganze Zahl von 0 bis 8 steht;
b) Zugabe einer Verbindung gemäß der Formel (**II**): 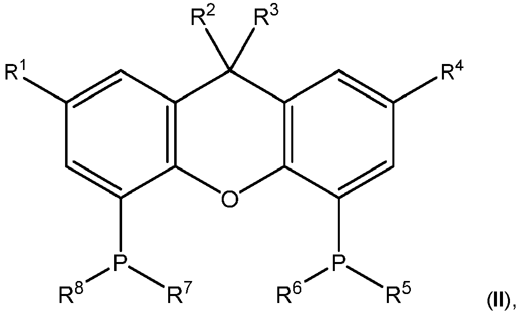 wobei R¹, R², R³, R⁴, R⁵, R⁶, R⁷, R⁸ ausgewählt sind aus: -H, -(C₁-C₁₂)-Alkyl, -(C₆-C₂₀)-Aryl;
   und für den Fall, dass R¹, R², R³, R⁴, R⁵, R⁶, R⁷, R⁸ für -(C₆-C₂₀)-Aryl stehen, kann der Arylring Substituenten aufweisen, welche ausgewählt sind aus: -(C₁-C₁₂)-Alkyl, -O-(C₁-C₁₂)-Alkyl;
c) Zugabe von PtI₂;
d) Zuführen von CO und H₂;
e) Erwärmen des Reaktionsgemisches aus a) bis d), wobei der Ester zu einer Verbindung gemäß Formel (**III**) umgesetzt wird:

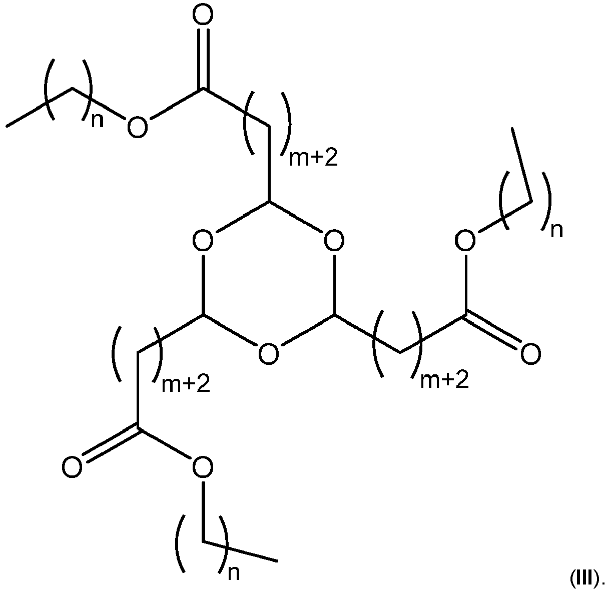

Hierbei können die Verfahrensschritte a) bis e) in beliebiger Reihenfolge erfolgen. Üblicherweise erfolgt die Zugabe von CO und H₂ jedoch, nachdem die Reaktionspartner in den Schritten a) bis c) vorgelegt wurden.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

In einer Variante des Verfahrens sind R², R³, R⁵, R⁶, R⁷, R⁸ ausgewählt aus: -(C₁-C₁₂)-Alkyl, -(C₆-C₂₀)-Aryl.

In einer Variante des Verfahrens stehen R⁵, R⁶, R⁷, R⁸ für -(C₆-C₂₀)-Aryl.

In einer Variante des Verfahrens stehen R⁵, R⁶, R⁷, R⁸ für -Ph

In einer Variante des Verfahrens stehen R² und R³ für -(C₁-C₁₂)-Alkyl.

In einer Variante des Verfahrens stehen R² und R³ für -CHs.

In einer Variante des Verfahrens stehen R¹ und R⁴ für -H.

In einer Variante des Verfahrens weisen die Verbindung (**II**) die Struktur (**2**) auf:

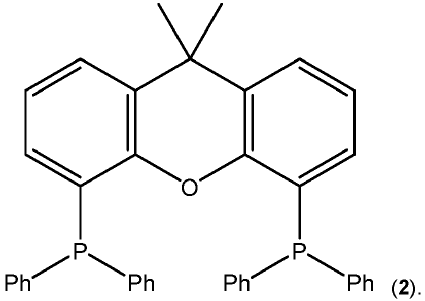

In einer Variante des Verfahrens steht m für eine ganze Zahl von 5 bis 9.

In einer Variante des Verfahrens steht m für 7.

In einer Variante des Verfahrens steht n für eine ganze Zahl von 0 bis 4 steht.

In einer Variante des Verfahrens steht n für 0.

In einer Variante des Verfahrens weist der Ester die Struktur (1) aufweist:

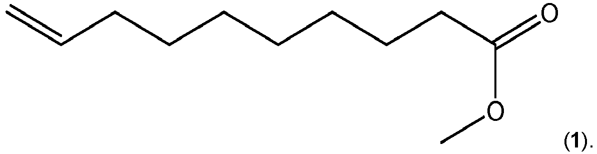

In einer Variante des Verfahrens erfolgt das Zuführen von CO und H₂ bei einem Druck in einem Bereich von 1 MPa (10 bar) bis 6 MPa (60 bar).

In einer Variante des Verfahrens erfolgt das Zuführen von CO und H₂ bei einem Druck in einem Bereich von 3 MPa (30 bar) bis 5 MPa (50 bar).

In einer Variante des Verfahrens erfolgt das Erwärmen auf eine Temperatur im Bereich von 40 °C bis 100 °C.

In einer Variante des Verfahrens erfolgt das Erwärmen auf eine Temperatur im Bereich von 50 °C bis 80 °C.

In einer Variante des Verfahrens umfasst dieses den zusätzlichen Verfahrensschritt d'):
d') Zugabe eines Lösungsmittels.

In einer Variante des Verfahrens ist das Lösungsmittel ausgewählt aus: THF, MTBE, DCM, ACN, Heptan, DMF, Toluol, Xylen, Mesitylen, Dibenzyltoluol.

In einer Variante des Verfahrens ist das Lösungsmittel Toluol.

Neben dem Verfahren wird auch eine Verbindung beansprucht.

Verbindung gemäß der Formel (IV): 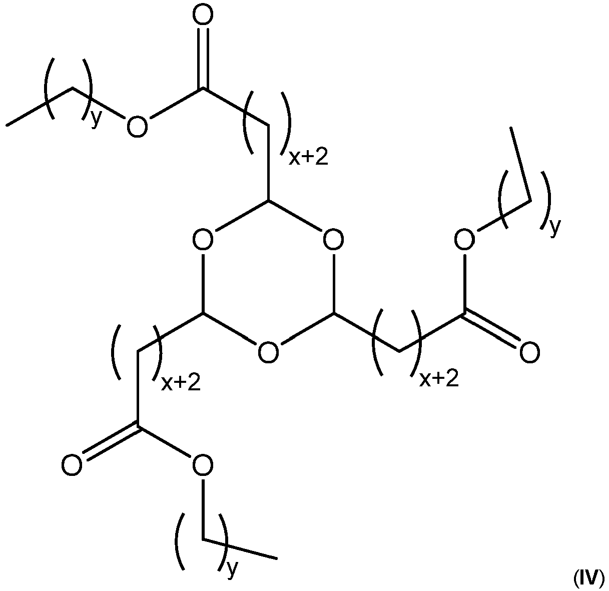 wobei x für eine ganze Zahl von 1 bis 10 steht und
y für eine ganze Zahl von 0 bis 8 steht.

In einer Ausführungsform steht x für eine ganze Zahl von 5 bis 9.

In einer Ausführungsform steht x für 7.

In einer Ausführungsform steht y für eine ganze Zahl von 0 bis 4.

In einer Ausführungsform steht y für 0.

In einer Ausführungsform weist die Verbindung gemäß der Formel (IV) die Struktur (4) auf:

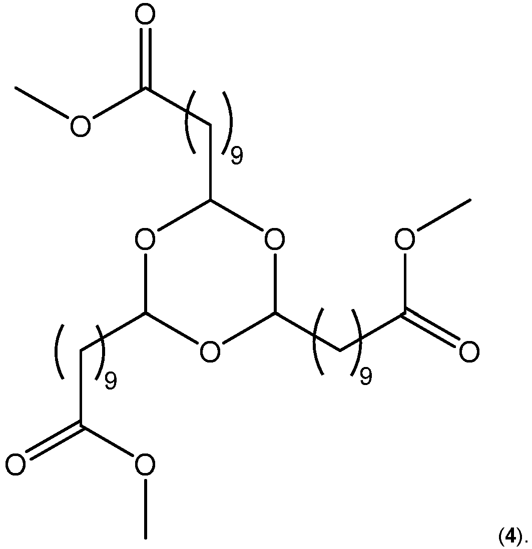

Im Folgenden soll die Erfindung anhand eines Ausführungsbeispiels näher erläutert werden.

### Synthese von Trimethyl-10,10',10"-(1,3,5-trioxan-2,4,6-triyl)tris(decanoat) (4)

150 ml (132 g) Methyldec-9-enoate (**1**), 100 ml absolutes Toluol, 320 mg Ptl₂ (0,1 mol % bezüglich (**1**)), 620 mg Xantphos (**2**) (0,15 mol % bezüglich (**1**)) werden in einem 450 ml Hochdruckautoklaven (Parr Instruments) versehen mit Rührung und elektronischem Druckaufnehmer unter Argon platziert. Es werden 40 bar Synthesegas (H₂:CO = 1:1) aufgepresst und die Reaktion bei 60 °C unter Rührung (>500min⁻¹) durchgeführt. Die Reaktionszeit beträgt 10 h. Der Gasverbrauch wird nachreguliert, so dass die Reaktion bei ca. 40 bar stattfindet. Nach 10 h wird die Reaktion abgebrochen, der Autoklav heruntergekühlt, das Gas abgelassen und der Autoklav 4 mal mit 30 bar Stickstoff gespült. Die Reaktionslösung wird in einen 500 ml Schlenkkolben überführt.

Es wird ein GC gemacht. Die GC-Ausbeute an Methyl-11-oxoundecanoat (3) beträgt: 98 %, (Selektivität: n: iso = 98,2: 1,8).

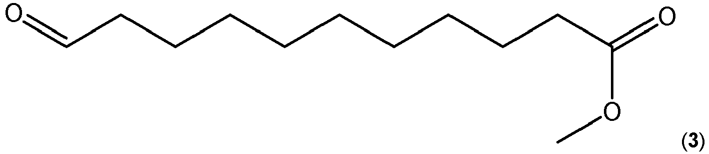

Anschliessend wird im Feinvakuum bei 10⁻³ Torr destilliert (K_{P} = 100 °C). Es resultiert eine farblose Flüssigkeit (146 g = 96 %).

Diese Flüssigkeit kristallisiert innerhalb von 24 h vollständig zu einem Feststoff durch, der als Trimethyl-10,10',10"-(1,3,5-trioxan-2,4,6-triyl)tris(decanoat) (**4**) mittels ¹H-, ¹³C-NMR- und MS -Analyse identifiziert wurde. Die Reinheit beträgt >99 %.

NMR (CDCl₃, 300 MHz):
¹H: 4,75 t(3 H, J_{HH} = 5,3 Hz), 3,59 s(9H), 2,23(6H, J_{HH} = 7,5 Hz), 1,6-1,5 m (12H) 1,38-1,16 m (36 H) ¹³C: 174,28 s ,101,65 s, 51,40 s, 34,39 s, 34,08 s, 29,38 s, 29,32 s, 29,20 s, 29,11 s, 24,93 s, 23,53 s

MS (70 ev, MZ (%)): 186(18), 171(44), 143(27), 139(65), 129(9), 121(17), 111(18), 98(36), 97(31), 87(78), 74(100), 69(43), 59(29), 57(17), 55(64).

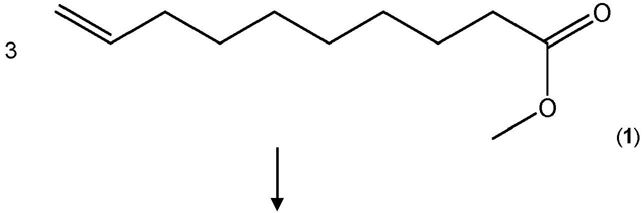

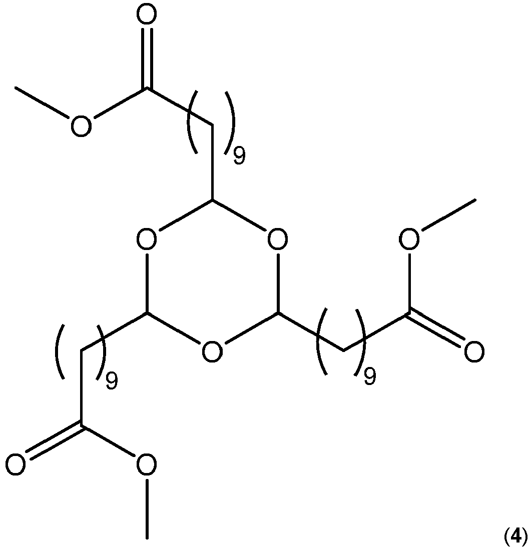

### Reaktionsbedingungen:

Ester (**1**), 0,1 mol% PtI₂, 0,15 mol% Xantphos (2), Lösungsmittel: Toluol, p(CO/H₂): 40 bar, T: 60 °C, t: 10 h.

Wie die Versuchsergebnisse zeigen, wird die Aufgabe durch das erfindungsgemäße Verfahren gelöst.

## Patentansprüche

1. Verfahren umfassend die Verfahrensschritte:
a) Vorlegen eines Esters gemäß der Formel (**I**): 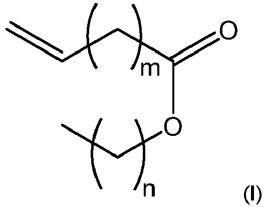 wobei m für eine ganze Zahl von 1 bis 10 steht und n für eine ganze Zahl von 0 bis 8 steht;
b) Zugabe einer Verbindung gemäß der Formel (**II**): 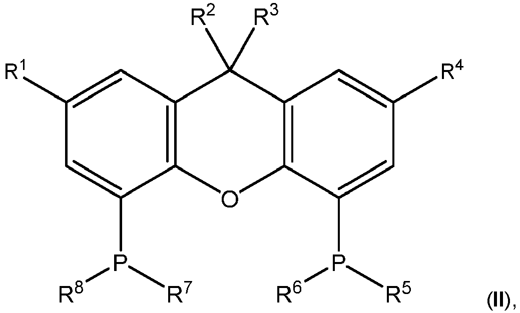 wobei R¹, R², R³, R⁴, R⁵, R⁶, R⁷, R⁸ ausgewählt sind aus: -H, -(C₁-C₁₂)-Alkyl, -(C₆-C₂₀)-Aryl; und für den Fall, dass R¹, R², R³, R⁴, R⁵, R⁶, R⁷, R⁸ für -(C₆-C₂₀)-Aryl stehen, kann der Arylring Substituenten aufweisen, welche ausgewählt sind aus: -(C₁-C₁₂)-Alkyl, -O-(C₁-C₁₂)-Alkyl;
c) Zugabe von PtI₂;
d) Zuführen von CO und H₂;
e) Erwärmen des Reaktionsgemisches aus a) bis d), wobei der Ester zu einer Verbindung gemäß Formel (**III**) umgesetzt wird:

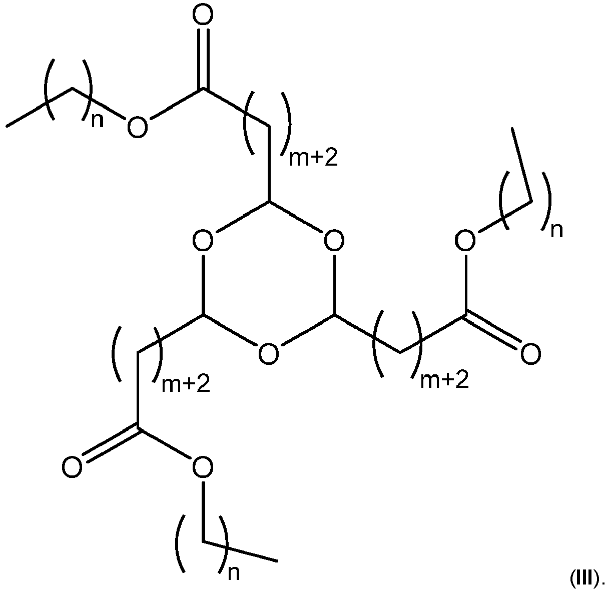

2. Verfahren nach Anspruch 1,
wobei R², R³, R⁵, R⁶, R⁷, R⁸ ausgewählt sind aus: -(C₁-C₁₂)-Alkyl, -(C₆-C₂₀)-Aryl.

3. Verfahren nach einem der Ansprüche 1 oder 2, wobei R⁵, R⁶, R⁷, R⁸ für -(C₆-C₂₀)-Aryl stehen.

4. Verfahren nach einem der Ansprüche 1 bis 3, wobei R² und R³ für -(C₁-C₁₂)-Alkyl stehen.

5. Verfahren nach einem der Ansprüche 1 bis 4, wobei R¹ und R⁴ für -H stehen.

6. Verfahren nach einem der Ansprüche 1 bis 5, wobei die Verbindung (**II**) die Struktur (**2**) aufweist:

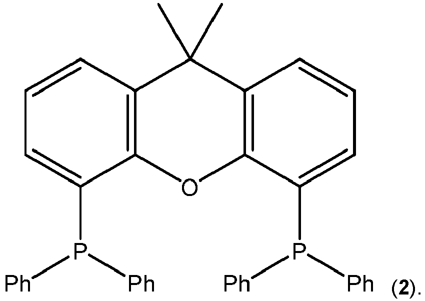

7. Verfahren nach einem der Ansprüche 1 bis 6, wobei m für eine ganze Zahl von 5 bis 9 steht.

8. Verfahren nach einem der Ansprüche 1 bis 7, wobei n für eine ganze Zahl von 0 bis 4 steht.

9. Verfahren nach einem der Ansprüche 1 bis 8, wobei der Ester die Struktur (**1**) aufweist:

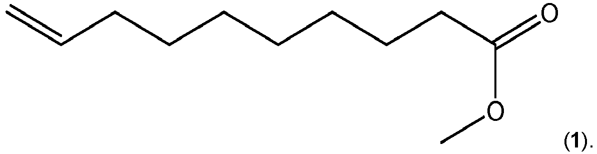

10. Verfahren nach einem der Ansprüche 1 bis **9**,
wobei das Zuführen von CO und H₂ bei einem Druck erfolgt in einem Bereich von 1 MPa (10 bar) bis 6 MPa (60 bar).

11. Verfahren nach einem der Ansprüche 1 bis 10,
wobei das Verfahren eine zusätzlichen Verfahrensschritt d') aufweist:
d') Zugabe eines Lösungsmittels.

12. Verbindung gemäß der Formel (**IV**): 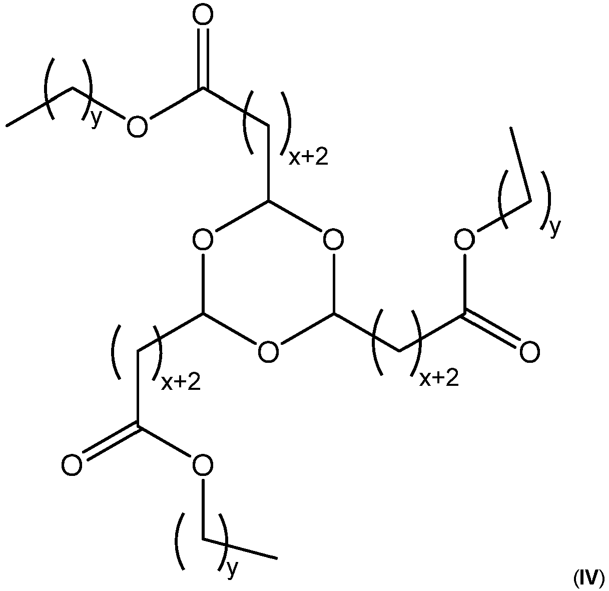 wobei x für eine ganze Zahl von 1 bis 10 steht und
y für eine ganze Zahl von 0 bis 8 steht.

13. Verbindung nach Anspruch 12,
wobei x für eine ganze Zahl von 5 bis 9 steht.

14. Verbindung nach einem der Ansprüche 12 oder 13,
wobei y für eine ganze Zahl von 0 bis 4 steht.

15. Verbindung nach einem der Ansprüche 12 bis 14,
wobei die Verbindung gemäß der Formel (**IV**) die Struktur (**4**) aufweist:

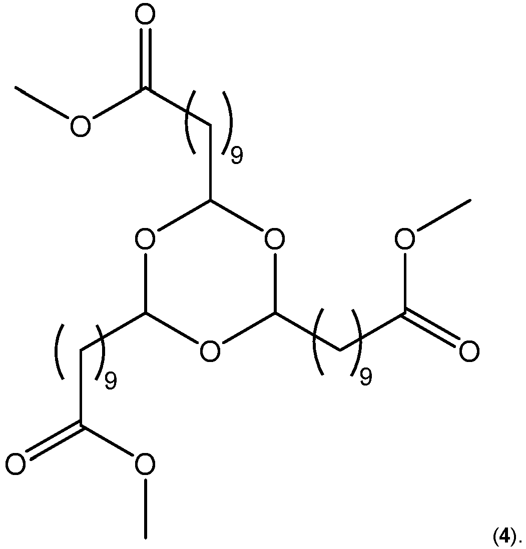

## Geänderte Patentansprüche

### Geänderte Patentansprüche gemäss Regel 137(2) EPÜ.

1. Verfahren umfassend die Verfahrensschritte:
a) Vorlegen eines Esters gemäß der Formel (I): 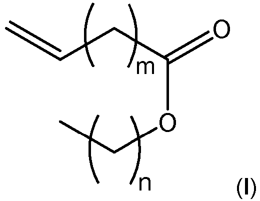
wobei m für eine ganze Zahl von 1 bis 10 steht und
n für eine ganze Zahl von 0 bis 8 steht;
b) Zugabe einer Verbindung gemäß der Formel (II): 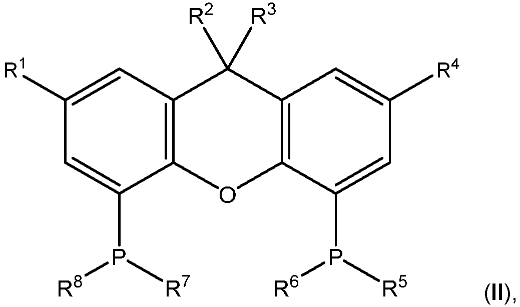
wobei R¹, R², R³, R⁴, R⁵, R⁶, R⁷, R⁸ ausgewählt sind aus: -H, -(C₁-C₁₂)-Alkyl, -(C₆-C₂₀)-Aryl;
und für den Fall, dass R¹, R², R³, R⁴, R⁵, R⁶, R⁷, R⁸ für -(C₆-C₂₀)-Aryl stehen, kann der Arylring Substituenten aufweisen, welche ausgewählt sind aus: -(C₁-C₁₂)-Alkyl, -O-(C₁-C₁₂)-Alkyl;
c) Zugabe von Ptl₂;
d) Zuführen von CO und H₂;
e) Erwärmen des Reaktionsgemisches aus a) bis d), wobei der Ester zu einer Verbindung gemäß Formel (**III**) umgesetzt wird:

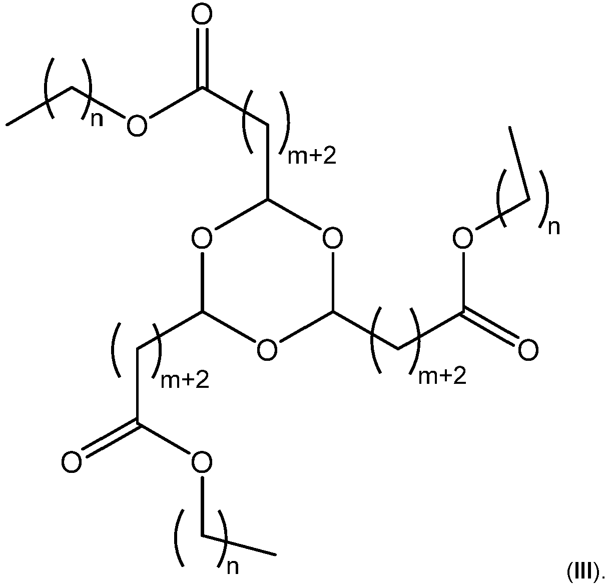

2. Verfahren nach Anspruch 1,
wobei R², R³, R⁵, R⁶, R⁷, R⁸ ausgewählt sind aus: -(C₁-C₁₂)-Alkyl, -(C₆-C₂₀)-Aryl.

3. Verfahren nach einem der Ansprüche 1 oder 2,
wobei R⁵, R⁶, R⁷, R⁸ für -(C₆-C₂₀)-Aryl stehen.

4. Verfahren nach einem der Ansprüche 1 bis 3,
wobei R² und R³ für -(C₁-C₁₂)-Alkyl stehen.

5. Verfahren nach einem der Ansprüche 1 bis 4,
wobei R¹ und R⁴ für -H stehen.

6. Verfahren nach einem der Ansprüche 1 bis 5,
wobei die Verbindung (II) die Struktur (2) aufweist:

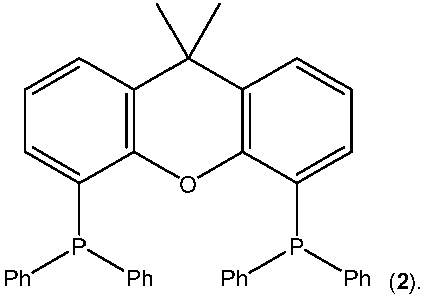

7. Verfahren nach einem der Ansprüche 1 bis 6,
wobei m für eine ganze Zahl von 5 bis 9 steht.

8. Verfahren nach einem der Ansprüche 1 bis 7,
wobei n für eine ganze Zahl von 0 bis 4 steht.

9. Verfahren nach einem der Ansprüche 1 bis 8,
wobei der Ester die Struktur (1) aufweist:

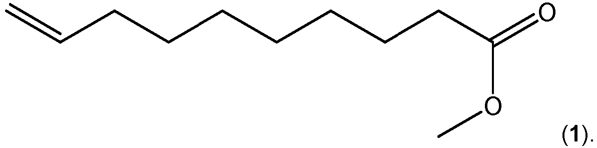

10. Verfahren nach einem der Ansprüche 1 bis 9,
wobei das Zuführen von CO und H₂ bei einem Druck erfolgt in einem Bereich von 1 MPa (10 bar) bis 6 MPa (60 bar).

11. Verfahren nach einem der Ansprüche 1 bis 10,
wobei das Verfahren eine zusätzlichen Verfahrensschritt d') aufweist:
d') Zugabe eines Lösungsmittels.
